Europäisches Patentamt

European Patent Office

Office européen des brevets

(19)

(11) Publication number: **0 202 470 B1**

(12) **EUROPEAN PATENT SPECIFICATION**

(45) Date of publication of patent specification: **03.06.92**

(21) Application number: **86105152.2**

(22) Date of filing: **15.04.86**

(51) Int. Cl.5: **C12N 15/00**, C12N 1/20, C07K 15/00, C12P 21/00, C12P 1/04, C12R 1/07, C12R 1/125

(54) **Cloning and expression of delta-endotoxin genes of bacillus thuringiensis in bacterial cells and products therefrom.**

(30) Priority: **20.05.85 US 736208**

(43) Date of publication of application:
**26.11.86 Bulletin 86/48**

(45) Publication of the grant of the patent:
**03.06.92 Bulletin 92/23**

(84) Designated Contracting States:
**BE DE FR IT**

(56) References cited:
**EP-A- 0 063 949**
**EP-A- 0 093 062**

(73) Proprietor: **ABBOTT LABORATORIES**
**14th Street and Sheridan Road North St**
**North Chicago, Illinois 60064(US)**

(72) Inventor: **Shivakumar, Annapur G.**
**1046 Cumberland Court**
**Vernon Hills Illinois 60061(US)**
Inventor: **Benson, Terry Anne**
**203 N. Lewis Avenue**
**Waukegan Illinois 60085(US)**
Inventor: **Gundling, Gerard James**
**153 Wildwood Road**
**Lake Forest Illinois 60045(US)**
Inventor: **Spear, Brian Blackburn**
**2333 Kenilworth Avenue**
**Wilmette Illinois 60091(US)**

(74) Representative: **Modiano, Guido et al**
**MODIANO, JOSIF, PISANTY & STAUB**
**Modiano & Associati Via Meravigli, 16**
**I-20123 Milano(IT)**

EP 0 202 470 B1

Proceedings of the National Academy of Sciences of the United States of America, vol. 79, n 19 October 1982 (Baltimore, USA) G.A. Held et al "Cloning and localization of the lepidopteran protoxin gene of Bacillus tnuringiensis subsp. kurstaki", pages 6065-6069

Proceedings of the National Academy of Sciences of the United States of America, vol. 78, n 5 May 1981 (Baltimore, USA) H.E. Schnepf et al "Cloning and expression fo Bacillusthuringiensis crystal protein gene in Escherichia coli", pages 2893-2897

The Embo Journal, vol. 1, n 5, 1982 (Heidelberg, Oxford) A. Klier et al "Cloning and expression ofthe crystal protein genes from Bacillus thuringiensis strain berliner 1715", pages 791-799

**Description**

BACKGROUND OF THE INVENTION

Field of the Invention

The present invention relates to the insecticidal δ-endotoxin protein crystals found in Bacillus subtilis and which correspond to those found in Bacillus thuringiensis, variety Kurstaki, to novel forms and compositions thereof, and to novel means and methods for their production.

Background Art

The disclosure hereof is intended to be read in conjunction with the references cited, which are set forth in the appended Bibliography.

Bacillus thuringiensis is a spore-forming organism closely related to Bacillus cereus. Its peculiarity, however, is the appearance of proteinaceous crystals upon sporulation. The crystals are bipyramidal in shape, account for up to 30% of the dry weight of the sporulated culture, and are known to be toxic to most lepidopteran and some dipteran species of insect.

Varieties of B. thuringiensis are distinguishable by their flagellar or "H" antigens. They can also be classified according to their crystal type. The two classifications overlap in that the same serovars may have more than one crystal type and different serovars may have the same crystal type. Different varieties display varying toxicities to specific insects. Among these varieties is B. thuringiensis, var. Kurstaki (also referred to as HD-1), which is a particularly potent isolate against lepidoptera.

The Kurstaki isolate has been widely used commercially as an insecticide against many agricultural pests such as cabbage loopers (Trichoplusia ni), tobacco budworms (Heliothis virescens) and tobacco horn worms (Manduca sexta), as well as forest pests such as gypsy moths (Lymantria dispar). The advantages of this isolate over chemical insecticides include being nontoxic to mammals and advantageous insects (Heimpel, 1971), having no induced resistance in target insects and being nonaccumulative in the environment. The disadvantages have been its specificity towards only a small number of insects and its low viability after application, as it is quite susceptible to weathering and sunlight (Cantwell & Franklin, 1966; Morris & McErlane, 1975).

The toxic moiety of the HD-1 crystal is termed δ-endotoxin. The δ-endotoxin must be digested by the target insect in order to impart its pathogenicity. The alkaline pH of the insect gut allows for the breakdown of crystal protein leading to gut paralysis and generalized toxemia. Neither intact nor digested-crystal is toxic when injected into the insect, but both are toxic when administered orally (Angus, 1964). There also may be interaction between the crystal and spore, enhancing the toxicity. The nature of this interaction is unknown. (Yamvrias, 1962; Burges, 1976).

The crystal protein requires a high pH and the presence of reducing agents to solubilize it. The denatured subunits of the crystal protein give molecular weights of about 130 and 134 thousand daltons. There is evidence of a minor separate protein, P2 (also called mosquito factor) in HD-1 that can be isolated separately from the crystal, has a molecular weight of about 65,000 daltons, and displays toxicity to both lepidoptera and diptera (Yamamoto, 1983).

Genetics of B. thuringiensis ("B.t.")

Evidence has been presented that there is more than one structural gene contributing to the formation of the B. thuringiensis crystal. Schnepf & Whiteley (1981) have cloned a crystal gene from the kurstaki strain which shows homology to 2 large B. thuringiensis plasmids of 30 and 47 megadaltons. Klier et al.-(1982) using the subspecies thuringiensis strain berliner 1715, have identified both a plasmid and a chromosomal copy of a crystal gene. In kurstaki, however, Klier's group found only a plasmid copy. Held et al. (1982) claim to have found both chromosomal and plasmid copies in Kurstaki.

Whiteley's group isolated a plasmid copy from a partial Sau3A library of plasmid DNA cloned into the E. coli plasmid vector pBR322. The recombinant plasmids were transformed into the E. coli strain HB101 and screened for inserts on the basis of immunoreactivity with antibody made to purified B.t. crystals. They isolated a clone that produced a 130,000 dalton protein, the same size as the crystal protein, and which displayed toxicity to tobacco horn worm. This clone has been partially sequenced and two transcriptional start sites have been noted (Wong et. al., 1982). Using RNA's synthesized in B.t. during sporulation, they located two adjacent start sites, one initiating transcription early in sporulation and the other during the

3

middle of sporulation. Only one RNA species was detected in the E. coli clone containing the gene. The crystal protein in E. coli was produced at all stages of growth, although at a low level. The first 8 amino acids coded by the gene's coding region match those at the amino terminus of the crystal protein. A potential ribosomal binding site was also located.

Whiteley's group also used a portion of this clone for probing gene libraries from other strains including Kurstaki, HD73 and sotto. HD73 makes a different crystal type and sotto has only 2 plasmids. Both of these libraries had clones producing 130,000 dalton proteins that cross reacted with antisera made to HD1 crystal protein and expressed toxicity to tobacco horn worms.

Held et al. (1982) cloned a B.t. total DNA library from EcoRI digestion into the phage vector Charon 4A. Using antibody detection a clone was identified, which, when subcloned into E. coli plasmid pBR328 and B. subtilis plasmid pHV33, produced antigen that cross-reacted with anti-crystal protein antibody. Certain fragments of the subclones hybridized to a 45,000 dalton B.t. plasmid. Other fragments hybridized to both plasmid and chromosomal DNA and one fragment hybridized to only chromosomal DNA, suggesting the original clone was chromosomal in origin. This clone showed toxicity to tobacco horn worms.

Klier et al. (1983) used labeled stable B.t. mRNA's that appeared coincident with sporulation to probe an E. coli library of total DNA from thuringiensis strain berliner 1715. Four positive clones were hybridized back to total B.t. RNA, and it was discovered that three of them hybridized to a 26s mRNA. This 26s mRNA was not present in vegetative or acrystalliferous mutants. This clone was determined to be a chromosomal gene copy and did not transcribe or express in E. coli. A 4.0 kilobase subclone of this gene transcribed in both E. coli and B. subtilis, but did not express.

Klier's group also obtained a plasmid gene copy transformed into E. coli strain HB101. This clone was a 14 kilobase BamH1 fragment from a 42 megadalton berliner 1715 plasmid. This clone precipitated the antibody against B.t. crystal protein and showed toxicity. Upon transfer into B. subtilis, this clone produced a protein of 130,000 daltons, but only during sporulation. Inclusions were seen in E. coli clones and sporulating B. subtilis, but no regular crystalline structure or pattern was visible when examined by electron microscopy.

Recently, Kronstad, Schnepf and Whiteley (1983) have reported the possible presence of multiple crystal protein genes in several strains of subspecies thuringiensis and Kurstaki. Hybridization of an intragenic restriction fragment from a cloned crystal protein gene to the restriction digests of plasmids and total cellular DNA revealed that all homology to the crystal protein gene was plasmid associated and that several of these strains contained multiple regions of homology.

Recombinant DNA Technology

The recently developed recombinant DNA technology has provided means by which genes can be isolated, induced to higher levels of activity, or altered in nucleotide sequence. Such procedures have been used for a wide range of genetic research experiments, and, increasingly, for the production of proteins of commercial interest. Despite widespread use of such procedures, the isolation and expression of each each gene remains an experimental problem. The specific molecular and biochemical requirements for the efficient synthesis of a protein, and its recovery in a biologically usable form, have not been reduced to general procedures . The biological peculiarities of each gene and its host environment continue to demand creativity and inventiveness on the part of the scientist.

Recombinant DNA procedures involve the assembly, in vitro, of DNA molecules from multiple sources into a single, chimeric molecule. The assembly typically requires the fragmentation of DNA with site-specific restriction nucleases and the joining of these DNA fragments at their ends with the enzyme DNA ligase. Selection for appropriate segments for ligation results in the desired recombined DNA molecules. In most cases, the resulting molecule will have a replication origin (so that the DNA can be duplicated in a host cell), a marker gene, often one specifying resistance to antibiotic, and the foreign gene whose protein product is desired.

In bacterial systems, such recombinant DNA molecules are introduced into cells and thereafter replicated within those cells as either bacteriophage or plasmids. Bacteriophage can be assembled in vitro, with the recombinant DNA molecule enclosed within the viral protein capsule. The recombinant DNA is then introduced into the bacterium and duplicated by the normal processes of viral infection. Plasmids are DNA molecules that contain origins of replication and thus can duplicate themselves during cell growth. Recombinant plasmids constructed in vitro can be introduced into bacteria by treatment of the cells to make them take up exogenous molecules. The bacteria thus transformed with recombinant plasmids are plated at low density. A single colony thus represents a clone of bacteria all containing the identical foreign gene.

The host cell into which foreign genes are introduced has a substantial effect on the expression of the foreign genes. Typically, the bacterial host used is E. coli. E. coli has been extensively characterized genetically, and readily takes up foreign DNA. However, Bacillus subtilis has been used with increasing Frequency as a host cell. B. subtilis will respond to a different set of regulatory signals for gene expression which, for some genes, can result in enhanced protein synthesis. B. subtilis has also been favored for some proteins because of its ability to secrete proteins into the medium and because of its lack of toxins.

The expression of foreign proteins in bacteria requires the activity of a collection of interacting mechanisms. RNA synthesis requires the presence of promoter sequences adjacent to the gene. In some instances, the promoters that are situated naturally next to a cloned gene can be utilized by the host gene expression machinery. In other instances, the vector plasmid or bacteriophage must furnish the promoters for expression of the inserted gene.

Production of a foreign protein within a bacterial cell requires not only RNA synthesis but also synthesis and accumulation of the encoded protein. Bacterial ribosomes, which carry out protein synthesis, recognize a short binding sequence in RNA which must be encoded by the gene. This ribosome binding site can be part of the inserted gene or can be part of the vector. In some instances the inserted gene is linked to a resident gene in the vector in such a way that a fused protein is made, utilizing the promoter and ribosome binding site of the vector and the amino acid coding sequence of the insert. The amount of protein synthesized can vary widely from gene to gene depending on the regulatory sequences employed and on other factors that are not understood. The fate of a foreign protein is also variable. It may accumulate in as inclusion or intracellular crystal, remain soluble in the cell, be secreted, or break down due to the activity of proteases.

BRIEF DESCRIPTION OF THE DRAWINGS

The numeral set forth below represent Figure numbers for the appended drawings.

1. Strategy for the construction of the recombinant plasmid pHBHE. Thick lines represent the DNA from the plasmid pUC9. The hatched area in the block represents the toxin gene from B.t. DNA.

2. The B.t. toxin gene expression in HBHE. SDS-polyacrylamide gel electropherogram of proteins (A) and corresponding protein immunoblot (B) are shown. Lanes 1 to 3 correspond to proteins resolved from HD1-Dipel , E. coli strain carrying pUC9 and E. coli strain HBHE, respectively. Lane 4 shows protein molecular weight standards. Sizes of marker proteins are indicated.

3. The insecticidal crystal formation in genetically engineered bacteria. Transmission electron micrograph of bacterial cell sections of ABS-5 (A), ABS-9 (B), HD-73 (C), ABS-55 (D), ABS-58 (E) and HBHE (F).

4. Screening of B. subtilis transformants by colony hybridization. The dark spots on the right side represent colonies that hybridized to a nick translated probe. Alignment of these spots to cells on the master plates shown on the left identified the toxin gene containing transformants.

5. Southern blot detection of B.t. toxin gene in the plasmid fractions of B. subtilis transformants. A. Rapid plasmid preparations of some transformants analyzed on a 0.8% agarose gel by electrophoresis. B. Autoradiogram of plasmids from the same gel that were transferred to a nitrocellulose paper and hybridized to a nick translated probe.

6. Strategy for the construction of recombinant plasmids pABS-5 and pABS-9. The hatched area in the block shows the toxin gene from B.t. DNA.

7. The B.t. toxin gene expression in B. subtilis transformants. SDS-polyacrylamide gel electropherogram (A) and corresponding protein immunoblot (B), are shown. Lane 1, E. coli clone HBHE; lane 2, molecular weight standards; lane 3, ABS-5 (PSL1 containing pABS-5); lane 4, ABS-58 (BD 224 containing pABS-5); lane 5, ABS-9 (PSL1 containing pABS-9); lane 6, ABS-57 (SpoOH strain carrying pABS-9); lane 7, ABS-11 (PSL1 containing the vector pBD64 only); lane 8, ABS-59 (PSL1 containing pABS-59); lane 9, molecular weight marker proteins.

8. Growth curve of HD1-Dipel (□ - □), ABS-5 (O - O) and HBHE (X---X), in Luria broth medium.

9. B.t. toxin gene expression in B. subtilis and HD1-Dipel at different periods of growth. SDS-polyacrylamide gel electropherogram (A) and corresponding protein immunoblot (B), are shown. Lanes 1 to 9 show proteins from B. subtilis clone ABS-5 at 3, 6, 9, 22, 31, 45, 50, 76 and 120 hours of growth repsectively and lanes 11-19 show proteins from HD1-Dipel at 3, 6, 9 22, 31, 45, 50, 76 and 120 hours of growth respectively. Lane 20 shows the molecular weight markers.

10. The toxin crystal formation in B. subtilis strain ABS-5. Electron micrographic sections of cells grown to 9 hrs. (A), 13 hrs. (B), 24 hrs. (C), 30 hrs. (D) and 36 hrs. (E).

11. The toxin crystal formation in B. thuringiensis strain HD1-Dipel . EM sections of cells grown to 24 hrs. (A), 30 hrs. (B) and 36 hrs. (C).

12. Strategy for the construction of the recombinant plasmid pHC45. Heavy line indicates the toxin gene.

13. B.t. toxin gene expression in B. subtilis and E. coli. SDS-polyacrylamide gel electropherograms (A) and corresponding protein immunoblot (B) are shown. Lane 1, Molecular weight markers; lanes 2 and 3, ABS-55 (PSL1 strain that contains the recombinant plasmid pABS-55); lane 4, BSW7 (TB1 strain that contains the recombinant plasmid pBSW7); lane 5, HC45 (TB1 strain that contains the plasmid pHC45); lane 6, TB1 strain that contains only the vector pUC9; lane 8, B.t strain HD1-Dipel .

14. Strategy for the construction of plasmid pBSW7. Heavy line indicates the toxin gene fragment from pHC45 and the hatched line indicates the gene Fragment from pHBHE.

15. Strategy for the construction of plasmid pABS-55. Heavy line indicates the toxin gene fragment from pHC45 and the hatched line indicates the gene fragment from pHBHE.

## SUMMARY OF THE INVENTION

The present invention arises from the discovery that the genes for the toxin crystal protein could be located on the endogenous plasmids of Bacillus thuringiensis, variety Kurstaki. This discovery enabled the production of the toxin crystals in Bacillus subtilis, using the recombinant DNA technology, by channeling the particular genes through Escherichia coli and to B. subtilis. Insect toxicity of the crystals was then demonstrated in laboratory tests

The crystal protein produced by Bacillus thuringiensis has been found to be toxic to the larvae of a number of lepidopteran insects. Abbott Laboratories has been marketing the crystal protein product under the trademark DIPEL, to be used as an insecticide. The present invention provides an improved method to manufacture the Bacillus thuringiensis crystal protein in another bacterial host species called Bacillus subtilis. Bacillus subtilis is a nonpathogenic soil organism that has been used for the production of several industrially important enzymes. The present invention further improves upon the existing method of production of the insecticide, in that the toxin protein synthesis in the new host species, Bacillus subtilis, occurs in all phases of growth. In the parent organism, Bacillus thuringiensis, the synthesis of the toxin starts only much later in the growth phase during sporulation. Thus, the present invention overcomes such growth phase limitations and is likely to cut down the cost of production of the insecticide.

## DETAILED DESCRIPTION OF THE INVENTION

Bacillus thuringiensis, variety Kurstaki (Dipel HD-1 strain) has been used for many years as the organism for the production of an insecticidal composition, sold by Abbott Laboratories under the trademark DIPEL. The bipyramidal crystals made up of the $\delta$-endotoxin proteins are a result of the expression of the genes found on plasmids in B.t. By application of recombinant DNA technology in accordance with the present invention, the relevant genes have been located and engineered in such a way that they can be moved into Escherichia coli and Bacillus subtilis, both of which do not normally possess such genes. The genetically engineered strains of E. coli and B. subtilis produce -endotoxin proteins that are toxic to caterpillars. Furthermore, the proteins assemble into crystalline structures. In the modified B. subtilis strains, the cloned DNA starts expressing $\delta$-endotoxin proteins in the vegetative phase; its expression is not restricted only to the sporulation stage as it is in the parent strain B. thuringiensis. This can provide a cost effective means of production of the insecticide.

### Host Cell Cultures and Vectors

The E. coli strains used for DNA cloning and expression allow the selection of appropriate markers, such as ampicillin resistance, which are conferred by the incorporation of a specific cloning vector. The plasmids used as vectors also contain markers, such as galactosidase activity, whose presence or absence indicates the insertion of foreign DNA into that marker gene. Strains such as JM83 or JM105 (Messing, 1981) and plasmids such as pUC8 and pUC9 (Viera and Messing, 1982) were found to be very useful in cloning B.t. DNA.

Bacteriophage lambda vectors such as lambda 1059 (Karn et al. 1980) were also used in the cloning of B.t. DNA. These vectors will only grow on P2 lysogens if foreign DNA has been incorporated into their genome, replacing a fragment containing red and gam genes.

The Bacillus subtilis strains used for cloning and expression are naturally sensitive to antibiolics. However, the introduction of specific plasmids into these sensitive strains confers resistance to specific antibiotics. The B. subtilis strain PSL1 (Ostroff and Pene, 1983) is a restriction minus modification minus, recE4 strain and claimed to exhibit stable transformation phenotype. The Bacillus strain BD224, which is another recombination deficient strain (recE4), and some sporulation deficient mutants, were also used.

6

The vector pBD64, a recombinant plasmid of pUB110 and pC194 conferring resistance to chloramphenicol and kanamycin, was used for cloning the B.t. toxin gene. Insertion of a foreign DNA segment into the unique BglII site of pBD64 inactivates the gene for kanamycin resistance.

## Methods Employed in Preferred Embodiments of the Invention

### 1. DNA preparation

B. thuringiensis DNA For cloning is isolated by an SDS-phenol procedure that yields both plasmid and chromosomal DNA. B. thuringiensis protoplasts are lysed with sodium dodecyl sulfate at 60°. The lysate is then deproteinized with phenol, followed by chloroform extraction and ethanol precipitation. The dissolved ethanol precipitate is treated with ribonuclease and pronase, followed by phenol extraction, chloroform extraction and ethanol precipitation.

### 2. Lambda cloning of B. thuringiensis DNA.

DNA of lambda 1059 (Bethesda Research Laboratories) cut with BamHI is ligated to BglII fragments of B. thuringiensis in a 1:1 molar ratio. Approximately 0.3/ mcg of the ligated mixture is packaged into phage particles using the "packagene" mixture purchased from BioTec. Recombinant phage are absorbed into E. coli Q358 (Bethesda Research Laboratories) which carries the P2 lysogen.

### 3. Gel electrophoresis of DNA.

DNA is separated by electrophoresis in gels of 0.75 to 1.2% agarose in 5mM sodium acetate, 2mM EDTA, and 40mM Tris at pH 7.6 at a voltage of 15 to 25 V overnight or 80-120 V for 1 to 4 hours.

### 4. Size separation of DNA fragments.

DNA fragments produced by digestion with restriction nucleases are separated by gel electrophoresis and visualized by ethidium bromide staining. Regions of the gel containing DNA molecules of the desired size are excised, placed in a microcentrifuge tube, and frozen at -70°C. After thawing, the agarose is crushed with a glass rod. Water is added to a total volume (of agarose and liquid) of 0.5 ml. The suspension is extracted twice with phenol, once with chloroform and precipitated with ethanol.

### 5. Preparation of competent cells of E. coli.

E. coli cells growing in LB medium at 37°C are spun down at a density of approximately $5 \times 10^7$ cells/ml and washed with 1/5 vol of (CTB) cold transformation buffer (45 mM $MnCl_2$, 60 mM $CaCl_2$, 100 mM $RuCl_2$, 40 mM KOAc, pH 6.2 and 15% sucrose, - filter sterilized). The pellet is resuspended in 1/40 volume of CTB, kept on ice for 10 minutes and 0.02 ml of DMSO is added to each 0.5 ml of resuspended cells and frozen at -70 C until they are ready to be used for transformation.

### 6. Restriction Digestion and E. coli Transformation

Insertion of foreign DNA into a plasmid vector is accomplished by cleaving the foreign and plasmid DNA with restriction enzymes and ligating the fragments together. About 1 mcg of DNA is cleaved with 1 to 10 units of restriction enzyme by incubating them together approximately for 1 hour at 37°C. An appropriate buffer, specified by the enzyme manufacturer, is included in the reaction mixture. The reaction volume is usually 20 microliters. Following the incubation, the DNA is either precipitated with isopropanol (when the DNA is to be run on a gel for restriction analysis) or phenol and chloroform extracted, then ethanol precipitated (when DNA is to be further treated). A restriction digest can produce either blunt or sticky ends. If it is necessary for sticky ends with 5' extensions to be made blunt, the cleaved DNA is incubated with free nucleotides and 5 units of T4 DNA polymerase I (Klenow fragment) for 30 minutes at 25°C.

Prior to ligation, the cleaved vector may be dephosphorylated to prevent self-ligation. This is done by heating it at 65°C for 15 minutes with bacterial alkaline phosphatase and the appropriate buffer, followed by phenol-chloroform extractions and ethanol precipitation. Ligations are carried out by combining DNA fragments and plasmid (a workable molar ratio is 4:1) in a 20 microliters volume containing between 2 units

7

(for sticky ends) and 10 units (for blunt ends) of ligase in the specified buffer. The mixture is kept at 20°C for several hours.

The plasmid constructions thus achieved are transformed into competent E. coli cells by mixing the DNA and cells and heating at 42°C for 1.5 minutes. A small amount of media is added and the cells are placed at 37°C for 1/2 to 1 hour before being plated on selective media. Plasmid is isolated from small cultures of selected colonies by alkaline hydrolysis (as described by Maniat's, Fritsch and Sambrook in "Molecular Cloning", Cold Spring Harbor, 1982, p. 368). The plasmid can then be analyzed by restriction digest and gel electrophoresis.

F. Immunological Detection of B.t. Crystal Proteins

Antibodies specific to the B.t. crystal proteins are produced in rabbits by standard procedures. Highly purified crystal proteins are used as the antigen. Renograffin purified crystals are solubilized in a SDS-urea sample buffer and the 130-135 kd proteins are resolved on SDS-PAGE. The protein bands are visualized with 3M sodium acetate and cut out. The gel slice is injected into rabbits to elicit an immunological response. The antibodies produced are specific For B.t. crystal proteins.

The production of B.t. crystal proteins by phage colonies and bacterial clones is detected with the rabbit antibodies. The proteins in the phage colonies are bound to a nitrocellulose filter placed directly on the culture plate. The proteins produced in bacterial clones are resolved by SDS-PAGE and then transferred electrophoretically to nitrocellulose sheets. The presence of B.t. crystal proteins is visualized using a double antibody technique. The nitrocellulose sheets and filters are incubated with immune rabbit serum which binds only to the immobilized B.t. crystal proteins. The nitrocellulose is then treated with goat-antirabbit IgG which is conjugated with horseradish peroxidase. The second antibody only binds to the rabbit antibody. The peroxidase reacts with a reagent, Bio-Rad HRP color development reagent, which produces a color reaction where the antibodies bind to the B.t crystal proteins on the nitrocellulose.

8. Synthetic Probes

Synthetic DNA probes are constructed manually using the phosphoramidite chemistry (Matteuci and Caruthers 1981) and purified by standard PAGE techniques. A probe which is complementary to the first fifteen coding bases of the B.t. crystal protein gene will specifically hybridize to cloned DNA containing that sequence.

9. Colony Hybridization

Bacterial transformants are spotted and grown on nitrocellulose filters placed on culture media. The cells are lysed by floating the filters on 0.5M NaOH and then neutralized with 1M Tris pH 7.4. The filters are then treated with 1.5M NaCl, 0.5M Tris (pH 7.4), prior to being baked at 80°C for one hour. Filters are first washed in 6X SSC and then washed in 50mM Tris (pH 8.0), 1M NaCl, 1mM EDTA and 0.1% SDS. The filters are prehybridized in 50mM iris pH 7.4, 1M NaCl, 10mM EDTA, 5X Denhardt's solution, 0.1% SDS, and calf thymus DNA, 0.1 mg/ml at 32°C for 3 hours. The filters are hybridized at 32°C in the same solution which contains the synthetic probe or a nick translated probe labeled with $^{32}$P-ATP. The probe is removed by washing the filters with 2X SSC, 0.1% SDS and 0.1% Na pyrophosphate. Clones which hybridize to the probe are located by autoradiography.

10. Insect Toxicity Tests

The clones are tested for toxicity by feeding cell extracts to insects, primarily Tricoplusia ni. Lyophilized phage lysates and lyophilized bacterial sonicates are mixed directly with an insect diet. Cell extracts, solubilized in a carbonate-DTT buffer, are measured for protein content and mixed with the diet to produce dilution curves to test the effectiveness of the protein. Newly molted 3rd instar T. ni are then placed on the solid diet and their growth is measured for one week. Toxic samples kill the insects in high concentration or stunt their growth in lower concentration. The relative toxicity of a sample is compared to known amounts of B.t. crystal protein in control tests.

11. Crystal Preparation

The parasporal crystals of B.t. are purified on linear preformed gradients of Renografin-76, sodium diatrizoate, according to the method of Sharpe et al., 1975. B.t. cultures are grown until sporulation is complete. The cells, spores, and crystals are collected by centrifugation and washed with water. The pellet is taken up in a small volume of water and sonicated. The mixture is pelleted through a solution of 50% Renografin and water. This process is repeated with the pellet which now contains predominately crystals and spores. The pellet is then taken up in water, sonicated, and layered on a 62 to 67% Renografin-76 linear gradient. The crystal band which is less dense than the spore band is collected and the process is repeated until pure crystals are obtained. The crystals are then washed several times with water to remove the Renografin and stored at 4°C.

12. Processing of Bacillus and E. coli Cells for EM Examination

Purified EM grade glutaraldehyde (Ted Pella Co., Tustin, Calif.) is added to cell suspensions (final conc. 0.2%), mixed and then incubated for 2 hours at room temperature. During the prefixation period 2% agar in water is first boiled and then cooled to 40°C in a water bath. The cell suspension is pelleted at 1500 Xg, the supernatant discarded and the tube containing the pellet placed in the water bath and allowed to equilibrate about 5 min at 40°C. Using a prewarmed Pasteur pipette, about 0.5 ml of the agar is transferred to the cell pellet and mixed so that the bacterial cells became suspended in the agar. A portion of the suspension is next drawn up into the tip of the pipette, allowed to cool briefly to room temperature and expelled into ice cold phosphate buffered 3% glutaraldehyde.

The resulting cylindrical-shaped congealed agar plugs containing the bacteria are further fixed in the glutaraldehyde for from 3 to 18 hours, washed in phosphate buffer and cut into small pieces. Samples are next post-fixed 1 hour in phosphate buffered 1% osmium tetroxide, dehydrated in a graded series of ethanols, and embedded in a Epon-Araldite embedding mixture. Following thin sectioning, samples are stained with uranyl acetate and lead citrate. The bacteria are examined by electron microscopy for presence of crystalline inclusions at magnifications ranging from 10-30,000x.

13. Transformation of B. subtilis

Competent cells are prepared as described by Dubnau and Davidoff-Abelson (1971). Plasmid transformation is performed as described by Contente and Dubnau (1979). Plasmid DNA and competent cells are incubated at 37°C for 30 min using 1-3 mcg DNA per ml. EGTA (1 mM) is used in the competent cell-DNA mixture. Selection for antibiotic resistance was carried out by the overlay method in TBAB agar after 90 min delay at 37°C to allow for expression. Selective concentrations of chloramphenicol and kanamycin are 5 mcg/ml.

14. B. subtilis Colony Hybridization

B. subtilis transformants are patched on TBAB agar plates containing chloramphenicol (5 mcg/ml) and grown by incubation at 30°C for 20-36 hours. A nitrocellulose filter is placed over the agar surface and is picked up carefully after 5 minutes and placed with the colony side up over 3 Whatman #1 filters soaked in 7 ml of lysozyme (2 mg/ml), then incubated at 37°C for 30 min. The nitrocellulose filters are then serially transferred to a petriplate containing denaturation buffer (0.5 M NaOH, 2.5 M NaCl) for 10 min and neutralization buffer (0.5 M Tris, 3 M NaCl) for 10 min. They are then briefly rinsed with 2x SSC and baked at 80°C for 2 hrs. The filters are soaked in 6 x SSC for 5 min and washed in 50 mM Tris-Cl, pH 8.0, 1 M NaCl, 1 mM EDTA and 0.1% SDS for 10 min. They are then prehybridized in a solution containing 50% formamide, 5x Denhardt's solution, 5x SSPE, 0.1% SDS and 125 ug/ml calf thymus DNA for 2 hrs at 42°C in a "seal-a'meal" bag. 15 ml of prehybridization mix is removed and a suitable nicktranslated probe is added and incubation continued at 42°C For 15 hrs. The filters are washed free of the probe with repeated washings with SSPE (0.18 M NaCl, 0.01 M Napp, 1 mM EDTA). Air dried filters are then exposed to Kodak X-omat film.

15. B.subtilis Extracts for toxicity testing

The clones identified as positive by Southern hybridization and by expression as identified by Western blotting are grown in 1 liter cultures in LB medium at 30°C for 2 days. After harvesting and washing the cellular pellet, it is sonicated for 3 minutes, shell freezed and lyophilized. This powdered preparation is tested for toxicity to insects.

General description of preferred embodiments.

The E. coli and B. subtilis organisms were engineered genetically to produce the insecticidal proteins according to the following protocol:

1. A plasmid library of B.t. genes was obtained by ligating enzyme restricted DNA fragments from B.t. to a universal cloning site of plasmid pUC9.

2. The library of B.t genes in pUC9 was introduced into competent cells of E. coli strain JM 83 and the transformants were picked by colony hybridization to a synthetic probe.

3. The 16.5 Kb plasmid pHB-1 from HB1 was trimmed down to a 8.2 kb plasmid named pHBHE.

4. The protein extract made from HBHE was shown to contain a 135,000 dalton protein that cross reacted with the B.t. toxin antibody.

5. The cell extracts of HBHE were shown to be toxic to caterpillars.

6. The toxin gene from pHBHE was ligated to a plasmid vector pBD64 and introduced into B. subtilis by transformation.

7. The B. subtilis transformants were screened by colony hybridization to a $^{32}$p-labeled fragment of pHBHE.

8. Transformants ABS-5 and ABS-9, shown to have the toxin gene inserted in opposing orientations produced a protein of 135,000 daltons that cross reacted with B.t toxin antibody in all growth phases.

9. Bipyramidal crystalline structures were found in ABS-5 and ABS-9.

10. Cell extracts of ABS-5 and ABS-9 were found to be toxic to caterpillars.

11. The toxin gene expression in B. subtilis was found to be independent of sporulation as confirmed by its expression in a sporulation deficient mutant of B. subtilis.

12. Another B.t. toxin gene that specifies a 130,000 dalton protein, was cloned in E. coli by first making a library of B.t. genes in the bacteriophage 1059 and subcloning into pUC9.

13. The E. coli transformant HC45 containing the plasmid pHC45 was shown to produce a protein of 130,000 daltons.

14. The toxin structural gene from pHC45 was fused with the promoter region of pHBHE and the recombinant plasmid pBSW7 was introduced into E. coli by transformation.

15. The E. coli strain BSW7 that contains the plasmid pBSW7, was shown to produce a protein of 130,000 daltons that cross reacted with the B.t. toxin antibody.

16. The composite gene from pBSW7 was cloned into pBD64 and introduced into B. subtilis by transformation.

17. The new B. subtilis transformant ABS-55 was found to produce a protein of 130,000 daltons that cross reacted with the B.t. toxin antibody.

18. The cell extracts of ABS-55 were found to be toxic to caterpillars.

19. The B. subtilis clone ABS-55 produced bipyramidal crystalline structures.

EXAMPLES

The following examples are intended to illustrate but not to limit the invention. Suitable strains of E. coli, B. subtilis and a bacteriophage λ•1059 have been used. The strains of B. subtilis which were resultant from the present invention have been deposited with the American Type Culture Collection under strain designations 53120, 53121 and 53122 , corresponding to the HBHE gene in plasmid pBD64 in B. subtilis PSLI, the Pst subclone of the gene coding for a 130,000 d protein in shuttle vector which includes pUC9 and pBD64 in B. subtilis PSL1, and a subclone of the gene coding for a 130,000 d protein with the HBHE promoter in pBD64 in the host B. subtilis PSL1, respectively.

Example I

Cloning of 135,000 dalton δ-endotoxin protein specifying genes from B. thuringiensis variety kurstaki HD-1 Dipel in E. coli.

E I A. Construction of a plasmid library of B.t. genes.

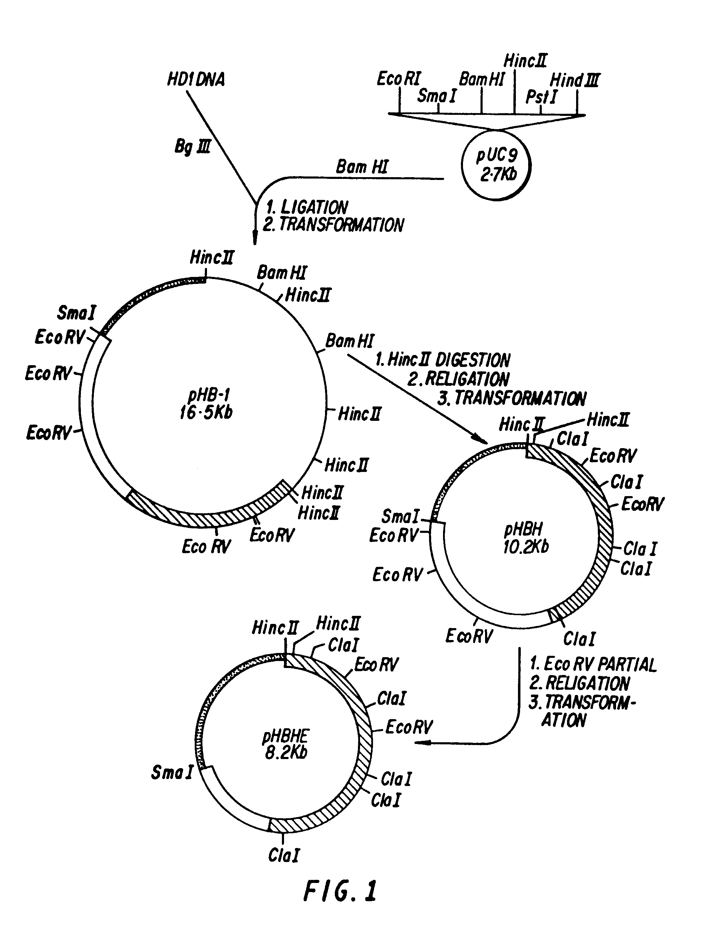

A plasmid library of HD-1 DNA was constructed with plasmid pUC9 and JM83 cells. Plasmid pUC9 was opened at the universal cloning site with restriction enzyme BamHI. The plasmid, 60 ng, was ligated with 2 mcg of BglII cut HD-1 DNA in a 20 microliter reaction mixture using 2 units of T4DNA ligase. The ligation mix was then used to transform competent E. coli JM83 cells. The DNA was mixed with 100 microliters of cells and the mixture was kept on ice for 10 minutes. The cells were heat shocked at 42°C for 1.5 minutes, cooled, mixed with 400 microliters of LB-broth and incubated at 37°C for 20 minutes. The cells were then plated on LB plates which contained 50 mcg/ml ampicillin and 4 mcg/ml Xgal. White colonies which indicated the presence of an insertion into pUC9, were picked and screened for B.t. crystal protein gene sequences by colony hybridization using a synthetic probe that is complementary to the first fifteen bases of the B.t. crystal protein gene. The resultant 16.5 kilobases-long recombinant plasmid was designated as pHB1. pHB1 was then digested with HincII and the fragments were circularized by ligation. Introduction of the ligation mixture into E. coli by transformation resulted in a collection of plasmids, one of which, pHBH, had lost a 6.3 Kb fragment upstream from the coding sequence. This plasmid was further partially digested with EcoRV and allowed to religate. When this mixture was transformed into E. coli, there was a further loss of a 2.0 kb fragment down stream from the coding sequence resulting in the new 8.2 kb plasmid designated as pHBHE. The sequence of constructions is shown in Figure 1. The bacterial strain that contained pHB HE was designated HBHE.

E I B. Expression of a 135,000 dalton B.t. $\delta$-endotoxin protein by the recombinant E. coli strain HBHE.

The E. coli strain HBHE and a strain that contains only pUC9 were grown for about 16 hours in LB medium containing ampicillin at 32°C. The protein extracts were prepared from the cells as described in METHODS and were separated by electrophoresis on a 8% SDS-polyacrylamide gel.

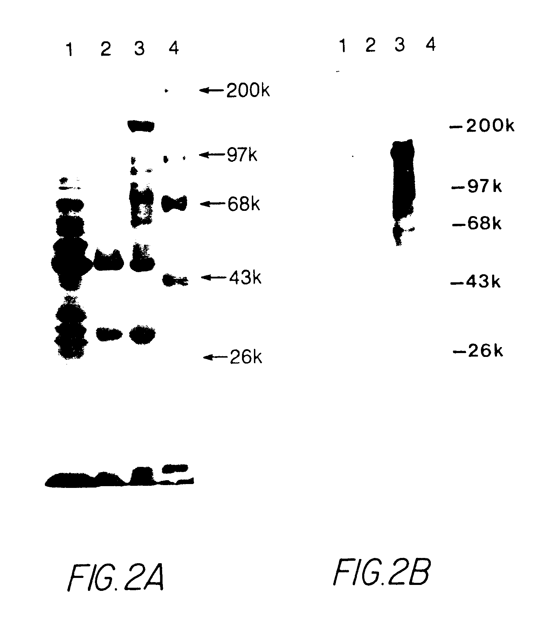

Results from Figure 2 show that the recombinant plasmid pHBHE expresses a gene encoding a protein of the size of about 135,000 daltons; and that this protein has the same mobility as that of the HD1 crystal protein. Lanes 1, 2 and 3 show the proteins from the parent B.t. strain HD-1, the E. coli strain with the vector pUC9, recombinant E. coli strain HBHE, respectively, separated on a 8% SDS-polyacrylamide gel. Lane 4 shows the prestained high molecular weight standards. The stained proteins are shown in Figure 2A, and a Western blot of a similar gel is shown in Figure 2B. The 135,000 dalton protein seems to react specifically with the B.t. toxin antibody.

E I C. The occurrence of a crystalline body in the E. coli recombinant strain HBHE.

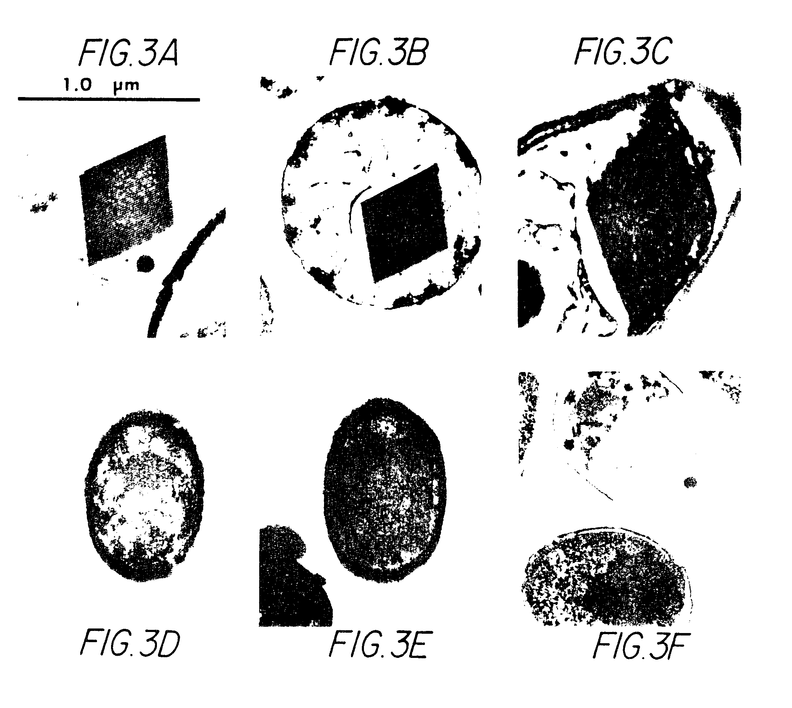

A growing culture of HBHE in LB medium was processed for electron microscopic examination as described in Methods, supra. Figure 3F shows the transmission electron microscopic picture of a section of the E. coli recombinant strain HBHE. Clearly, the recombinant E. coli strain forms a crystal, although not of a similar profile as in the parent B.t. strain (Fig. 3C). This was not evident in the strains containing the vector only.

E I D. Toxicity assay of the extracts from the recombinant DNA carrying E. coli strain HBHE.

Toxicity testing was conducted as described in Methods. At 1 mg/ml concentration of cell extract powder in the diet, all the 12 Trichoplasia ni 3rd instar larvae were found dead in 4 days. At 0.25 mg/ml, 9 out of 12 T.ni larvae were found dead in 6 days, and 3 were found to be very stunted.

Summarizing the results from Example I, the recombinant strain of E. coli, HBHE produces a 135,000 dalton protein which forms a semidefined crystal and reacts with the specific B.t. antibody. The plasmid vector pUC9, however, does not produce a protein that reacts with the B.t. antibody, and does not have a crystal showing that the B.t. insert in the vector results in production of the toxin crystal protein. The fact that the extracts from the strain containing the vector pUC9 only does not have any toxic effects on caterpillars, but the HBHE extract is toxic, shows that the new protein that is expressed in HBHE is toxic to caterpillars.

Example II

Cloning of the $\delta$ -endotoxin gene in Bacillus subtilis.

Here is described the isolation of the B.t. toxin gene from the E. coli recombinant plasmid pHBHE described in Example I, its introduction into Bacillus subtilis and its expression in B. subtilis.

E II A. Construction of the recombinant plasmid carrying the $\delta$-endotoxin gene of B. thuringiensis, variety kurstaki, for propagation in B. subtilis.

The recombinant plasmid pHBHE described in Example I was used as the source of B.t. $\delta$ -endotoxin gene. The vector was pBD64 (Gryczan et. al., 1980), containing the genes for resistance to chloramphenicol and kanamycin in B. subtilis. A cloning event in the unique BglII site inactivates kanamycin resistance. The pHBHE was cut with the restriction endonucleases SmaI and HincII, resulting in the generation of two blunt ended fragments of 5.5 and 2.7 kilobases. The vector pBD64 was linearized at its unique BglII site by digesting with BglII enzyme. The sticky ends generated were filled in using Klenow fragment of DNA polymerase I.

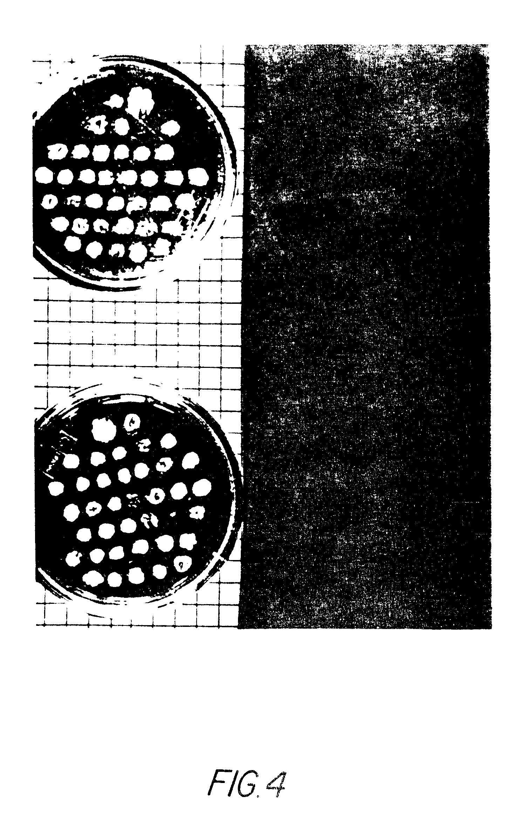

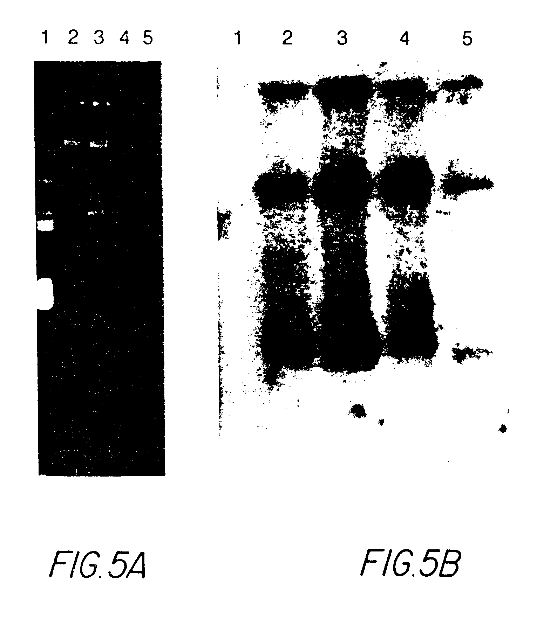

1 mcg of linearized and blunted pBD64 was then mixed with about 4 mcg of HincII-SmaI double digest of pHBHE and joined with ligase. Extent of ligation was monitored by agarose gel electrophoresis. The ligated DNA was then introduced into the competent cells of B. subtilis strain PSLI by transformation. The resultant chloramphenicol resistant colonies were screened for the presence of the $\delta$ -endotoxin gene by colony hybridizationusing $^{32}$P-labeled 5.5 kb fragment obtained from a double digestion of pHBHE with HincII and SmaI. Figure 4 is presented to illustrate a typical experiment of this sort. The dark regions on the x-ray film shown in the right side of the figure represent the colonies containing DNA that hybridized to the probe. These were aligned with the colonies on the master colony plate to identify the positives. The colonies identified as positives were streaked for single colonies on rich media Agar plates (TBAB) containing 5 mcg/ml chloramphenicol. Rapid plasmid preparations were made from some of these positives and the presence of B.t. toxin gene insert in them was confirmed by Southern hybridization. Figure 5A shows the plasmids electrophoresed on a 0.8% agarose gel and 5B shows an autoradiogram of the southern blot of the same gel. Lane 1 shows the vector pBD64 used for cloning and Lanes 2-5 show some of the transformants. It is clear that the transformants contain the B.t. toxin gene insert while the vector itself does not.

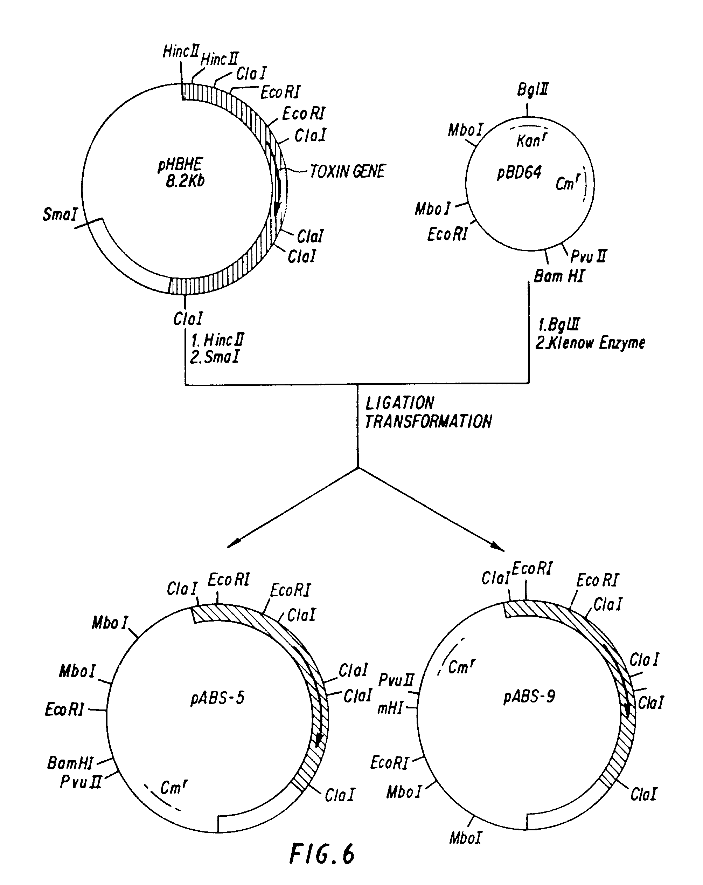

The mapping of the recombinant plasmids from some of these transformants by restriction enzymes indicated that the insert could be present in either orientation. One of them, present in orientation A, was designated as pABS-5 and the other in opposite orientation was designated as pABS-9. The sequence of construction events is shown in Figure 6.

Another recombinant plasmid pABS-59 was constructed by ligating the HincII-SmaI double digest of pHBHE to the unique PvuII site of pBD64, transforming into competent PSLI cells and screening by colony hybridization and colony immuneblotting.

E II B. Expression of $\delta$ -endotoxin by genetically engineered strains of B. subtilis.

The strains containing recombinant plasmids pABS-5, pABS-9, and pABS-59 are designated as ABS-5, ABS-9 and ABS-59, respectively. ABS-5, ABS-9 and ABS-59 were grown in Luria broth medium containing 5 mcg/ml of chloramphenicol for 16 hours at 30°C in a shaking incubator. The cells were spun and washed once in buffer A (60 mM NH$_4$Cl, 20 mM Tris-HCl, pH 7.5, 1 mM EDTA, 6 mM $\beta$ -mercaptoethanol, 2 mm PMSF, 10% glycerol and 1 M KCl) and twice in TESP buffer (30 mM Tris-Hcl, pH 7.6, 5 mM EDTA, 50 mM NaCl, 2 mM PMSF.) The cells were lysed in 1 mg/ml lysozyme in TESP, mixed with equal volume of 2x sample buffer (12 M urea, 5% SDS, 2% $\beta$ -mercaptoethanol, 0.25 M Tris-HCl, pH 6.8), boiled for 10 min cooled and spun. The clear supernatant was analyzed for proteins by SDS-polyacrylamide gel electrophoresis.

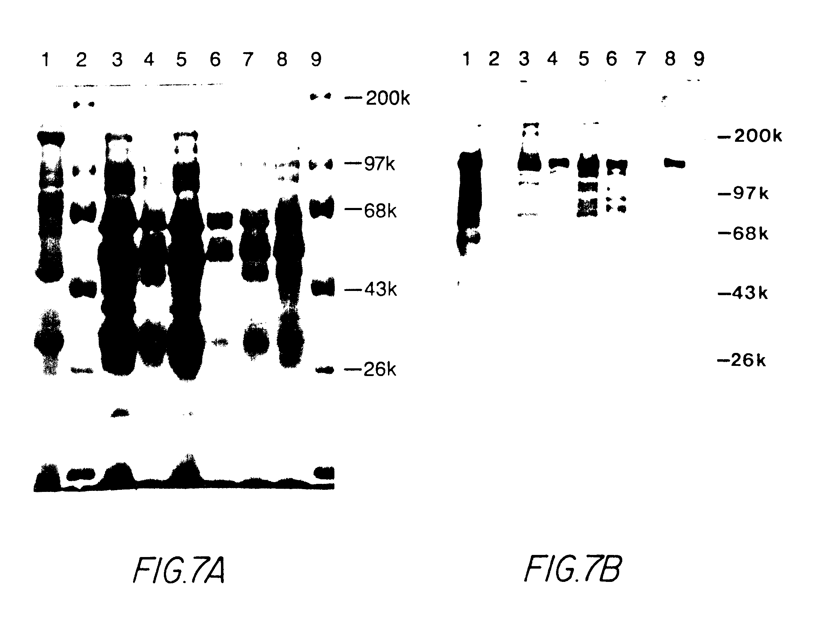

Figure 7A shows the proteins separated and stained on a 8% polyacrylamide gel. Figure 7B shows a western blotting of the proteins.

It is clear from the figure that strains ABS-5, ABS-9 and ABS-59 (Lanes 3, 5 and 8) do express a protein of about 135,000 daltons and that this protein reacts specifically with the antibody for B.t. crystal protein. Lanes 1 and 7 show proteins from the E. coli clone pHBHE and the PSL strain containing the vector pBD64 only. Neither the parent strain PSL1 nor the strain harboring only the vector pBD64 express proteins that react with the antibody.

The formation of well defined bipyramidal crystalline structures by ABS-5 and ABS-9 were noticed by transmission electron micrography and are shown in Figures 3A and 3B.

The following facts also indicate that the B.t. toxin gene cloned in B. subtilis uses its own promoter for expression of the 135,000 dalton protein.

1. pABS-5 and pABS-9, the two recombinant plasmids in which the toxin gene is inserted in opposite orientations, express a protein of 135,000 daltons.

2. pABS-59, which has the toxin gene inserted into a unique pvuII site located away from both kanamycin and chloramphenicol resistant genes of pBD64 vector, also express a similar sized protein.

E II C. Expression of $\delta$ -endotoxin in ABS-5 at different stages of growth.

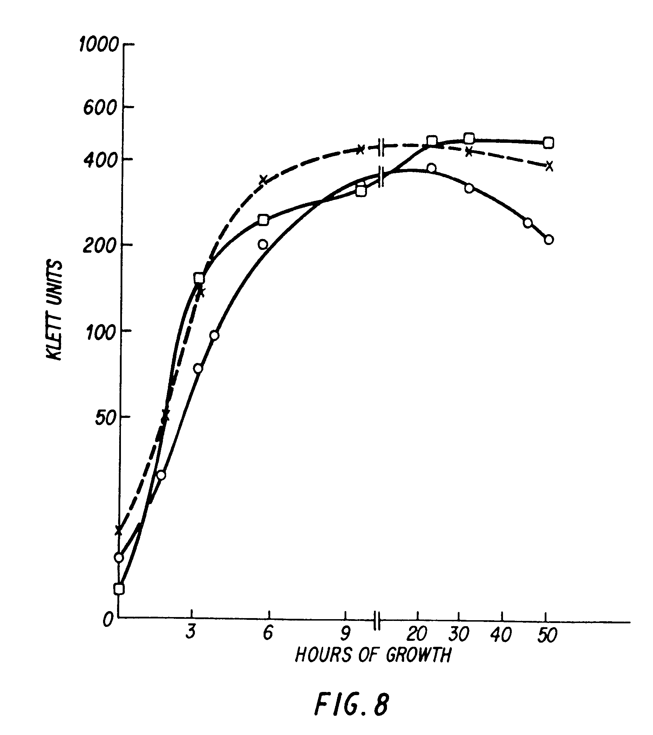

ABS-5 was grown overnight (about 16 hours) at 30°C in LB medium containing 5 m c/ml chloramphenicol. A culture of HD-1 (Dipel) was also grown in LB medium. Aliquots were diluted 100 fold into their respective fresh cultures in the morning and growth was monitored in a Klett colorimeter. Aliquots were removed at regular intervals from both cultures, extracts were made as described in Example EIIB and the proteins were separated by 8% SDS-polyacrylamide gel electrophoresis. Figure 8 shows a growth curve of the two cultures.

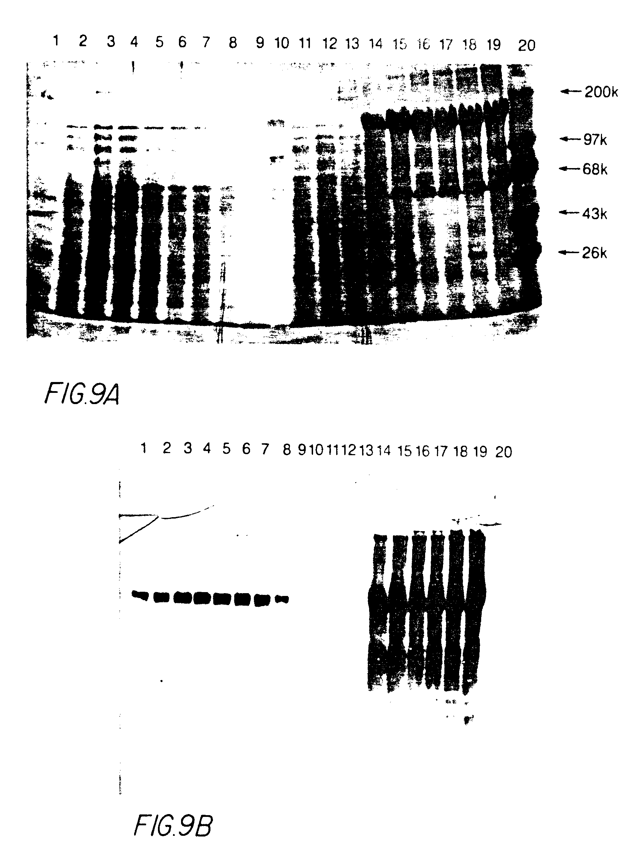

Figure 9A shows the stained proteins and Figure 9B shows a western blotting of the proteins.

It is clear from the figure (see figure legends for description), that the B. subtilis strain starts expression early in vegetative phase while the HD-1 (Dipel) cells clearly do not start expression until sporulation. Lanes 1 to 9 show proteins from the B. subtilis clone ABS-5 and Lanes 11 tO 19 show the proteins from the parent B.t. strains HDI-Dipel.

E II D. Electron Microscopic Examination.

The ABS-5 strain was grown in LB medium containing 5 mcg/ml chloramphenicol, overnight (16 hours)

EP 0 202 470 B1

at 30°C and subcultured by dilution 100 fold into fresh medium in the morning. Aliquots were removed at regular intervals and processed for transmission electron microscopy as described in Methods, supra.

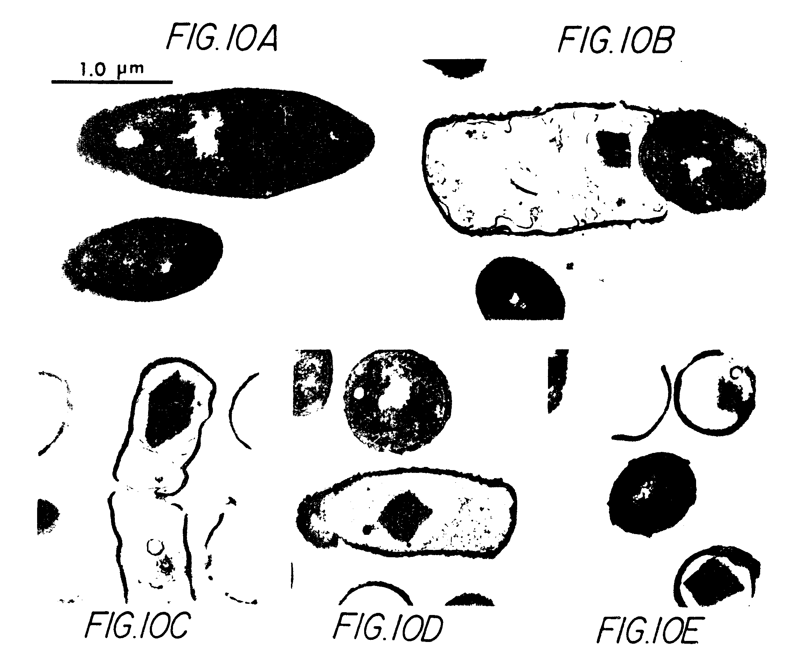

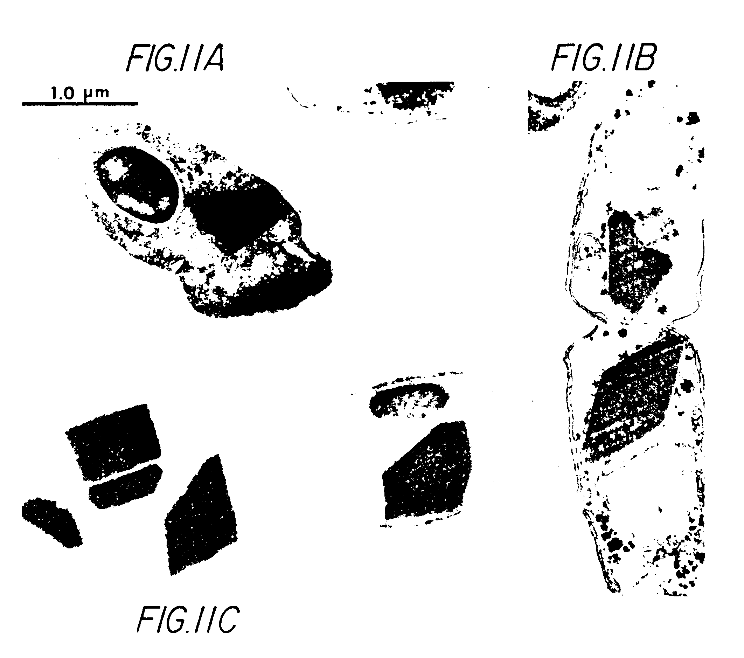

Figure 10 shows some electron microscopic sections of cultures of ABS-5 at different stages of growth. For comparison, HD-1 (Dipel) strain was also grown in LB medium, and aliquots removed at different stages of growth were processed similarly for transmission electron microscopy. Figure 11 shows EM sections of B.t. at different stages (see figure legends for description).

The appearance of small crystalline structures were apparent at as early as 13 hours in B. subtilis clones, while in B.t., clearly identifiable crystals seemed to appear after about 20 hours of growth.

E II E. Expression of the recombinant plasmid pABS-5 in a recombination deficient mutant of B. subtilis.

In order to show that the stability of the recombinant plasmid and expression of the insecticidal protein in the form of crystals is not due to the property of the strain PSLI, it was necessary to move the recombinant plasmid into another strain with a different background.

The B. subtilis strain BD 224 was provided by the Public Health Research Institute of the City of New York. It is a recombination deficient mutant and its markers are trpC2 recE4 thr-5.

BD 224 cells were made competent by the usual procedure as described in Methods, supra. The recombinant plasmid pABS-5 was introduced into the competent cells of BD224 by transformation. The transformants were selected for resistance to chloramphenicol. The plasmid DNA isolated from the transformants was found to be the same as pABS-5. This strain was designated as ABS-58 Cell extracts were made for protein analyses from growing cultures of ABS-58 in LB medium, and proteins were separated by electrophoresis on a 8% SDS-polyacrylamide gel. The stained proteins are shown in lane 4 of figure 7A and a western blot is shown in lane 4 of figure 7B. It is clear From this that ABS-58 expresses a protein of similar size as in B.t. and that it cross- reacts with the antibody for B.t. crystal protein.

E II F. Expression of the recombinant plasmid pABS-9 in a sporulation deficient mutant of B. subtilis.

To clarify that the toxin gene cloned in B. subtilis is indeed independent of sporulation it was essential to test its expression in a nonsporulating strain of B. subtilis.

The B. subtilis strain IS 160 was provided by the Public Health Research Institute of the City of New York. This strain is an early sporulation mutant and has the markers trpC2 pheA SpoOH15.

Competent cells of IS 160 were prepared as described in the Methods section, supra. pABS-9 recombinant plasmid was introduced into these competent cells by transformation. Transformants were selected for chloramphenicol resistance. The plasmid DNA isolated was found to be the same as in ABS-9. This nonsporulating strain containing the plasmid pABS-9 is designated as ABS-57.

Cell extracts were made from ABS-57 and analyzed For proteins by electrophoresis on a 8% SDS-polyacrylamide gel. The stained proteins are shown in Lane 6 of Figure 7A and a western blot of the same proteins is shown in lane 6 of Figure 7B. It is clear from the figure that the plasmid pABS-9 in ABS-57 does express a protein of about 135,000 daltons which is immunologically reactive with the antibody for the crystal protein. Thus, sporulation is unnecessary for expression of δ -endotoxin in B. Subtilis.

E II G. Expression of the reco binant plasmid pABS-9 in a root colonizing B. subtilis strain A-13.

A-13 is a B. subtilis strain that was isolated from Sclerotium Rolsii in Australia and it has been used commercially for treatment of peanut seeds A-13 is known to colonize the peanut roots and increase seedling emergence and reduce root pathology.

The recombinant plasmid pABS-9 was introduced into A-13 by transduction using a generalized transducing phage AR9 (Belyaeva and Azizbekyan, 1968). Selection was for chloramphenicaol using 5 mg/ml. The plasmid DNA isolated from these transductants were found tob be similar to pABS-9. This root colonizing B. subtilis strain A-13 containing pABS-9 is designated as ABS-65.

Cell extracts made from ABS-65 were analyzed for proteins by electrophores on a 8% SDS-polyacrylamide gel and the expression of a 135,000 dalton δ-endotoxin protein was confirmed by Western blotting analysis.

E II H. Cloning and expression of recombinant plasmids pABS-60 and pABS-61 in B. subtilis.

Two other recombinant plasmids were constructed using another B. subtilis plasmid pC194 (Horinswich and Weisblum, 1982). This plasmid has two unique cloning sites HindIII and HaeIII.

1 mg of pC194 plasmid DNA linearized with either HindIII or HaeIII was mixed with 4 µg of HincII-SmaI double digest of pHBHe (See E II A) and joined with T4 DNA ligase. Transformation, selection and identification of the transformants was by the same procedure outlined in E II A.

Toxin gene (HincII-SmaI Fragment of pHBHE) inserted into the HindIII site of pC194 in designated as pABS-60 and one that is inserted into the HaeIII site is designed as pABS-61 and the strains that contain these plasmids are designated as ABS-60 and ABS-61 cell extracts of these strains revealed a 135,000 dalton protein that cross reacted with the B.t. toxin antibody the level of expression was found to be similar to those from pABS-5 and pABS-9.

13

Example III

Cloning of 130,000 dalton δ -endotoxin protein specifying genes from B. thuringiensis variety kurstaki HD1-Dipel in E. coli.

E III A. Construction of lambda 1059 library of B.t. genes and cloning of a toxin gene in E. coli

A gene from the HD1-Dipel strain of B.t. that encodes the 130,000 d toxic protein was cloned in bacteriophage lambda 1059. B.t. DNA was cut with BglII and mixed with lambda 1059 DNA that had been cut with BamHI. T4 DNA ligase (40 units) was added, with reaction buffer to a final volume of 20 microliters. The ligation reaction was carried out overnight at 16°C. One fourth of the ligated DNA solution was added to 50 microliters of lambda packaging extract (Pakagene; Biotec) and the mixture was held at 22°C for 2 hours. One ml of SM buffer was added along with 25 m l of chloroform as a preservative.

A volume of 0.1 ml of a $10^{-1}$ dilution of the packaged recombinant phage was mixed with E. coli Q359 in soft agar and plated on each of five NZA plates. A total of 3.5 x $10^3$ plaques were obtained. Phage were then transferred to 82 mm nitrocellulose filters. Filters were applied onto the surface of the agar and allowed to bind phage for 2 hours at 22°. Filters with bound phage were soaked in 3% gelatin solution and then in a solution containing rabbit antibody against mixed 135,000 d and 130,000 d B.t. crystal proteins. Bound antibody was detected by binding goat anti-rabbit antibody conjugated with horseradish peroxidase and then developing with immunoblot (Bio-Rad) color reagent. Plaques of recombinant virus that contained and expressed B.t. crystal protein appeared purple on the filter. Plaques lining up with positives on the filter were picked from the agar plate, plaque purified, and stored in SM buffer with chloroform.

DNA was isolated from the recombinant phage lambda S4B, which has a strong immunoblot reaction, and partially digested with the restriction enzyme Sau3A. The DNA fragments were ligated into the plasmid pUC9 which had been cut with BamHI. The resulting recombinant plasmids were then screened by an immunoblot assay. One of the positive clones, named LSS2, contained 8.3 kb of B.t. DNA, and encoded the 130 kd B.t. crystal protein. A fragment of the LSS2 insert DNA, generated by digestion with restriction nuclease HincII, was subcloned into pUC9 also cut with HincII. This subclone codes for the 130 kd protein and is named pHC45.

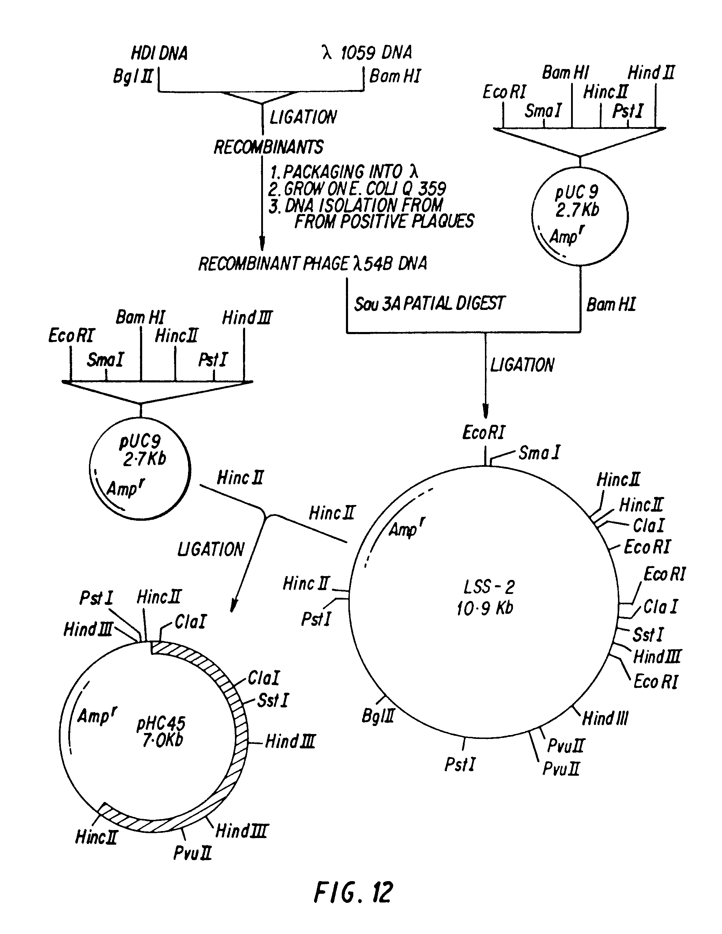

The sequence of events leading to the construction of pHC45 is shown in figure 12.

E III B. Expression of a 130,000 dalton polypeptide by pHC45.

The E. coli strain HC45 which contains pHC45 was grown in LB medium for 22 hours, and processed for protein analyzed as described in Methods, supra.

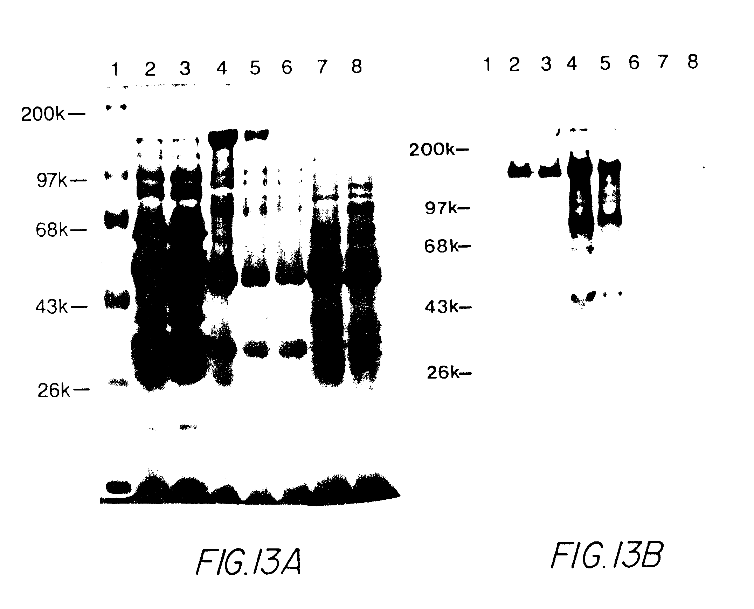

Results from Figure 13 confirm that the recombinant plasmid pHC45 does express in E. coli a protein of 130,000 daltons, the size of the smaller of the toxin proteins in the B.t. Dipel strain.

Lane 6 in Figure 13A shows the Coomassie blue stained proteins expressed by the vector pUC9 alone and lane 5 shows the proteins expressed by pHC45. Lanes 6 and 5 in figure 13b show a western blot of the proteins from pUC9 and pHC45. It is clearly seen that the pHC45 reacts specifically with the antibody to B.t. crystal protein.

Example IV

Cloning in E. coli of a composite plasmid containing the regulatory region from pHBHE and the structural gene from pHC45.

E IV A. Construction of a recombinant plasmid pBSW7.

The recombinant plasmid pHBHE described in Example I was used as a source of the promoter region and the recombinant plasmid pHC45 described in Example II, E II A, was used as the source of the toxin structural gene for the construction of a new recombinant plasmid pBSW7.

The plasmid pHBHE was digested with the restriction enzyme ClaI and dephosphorylated using bacterial alkaline phosphatase. It was then treated with a second enzyme SmaI. The 3.0 kb fragment consisting of the pUC 9 portion along with the promoter region between HincII and ClaI sites of the toxin gene insert was isolated and purified from an agarose gel. The plasmid pHC45 was cut with PstI and SmaI and then partially digested with the restriction enzyme ClaI. The fragments were separated by electrophoresis on an agarose gel and a 3.6 kb fragment was isolated and purified. Identity of both the 3.0 kb and 3.6 kb fragments were checked by restriction analyses. These were then mixed and ligated in presence of T4 DNA ligase.

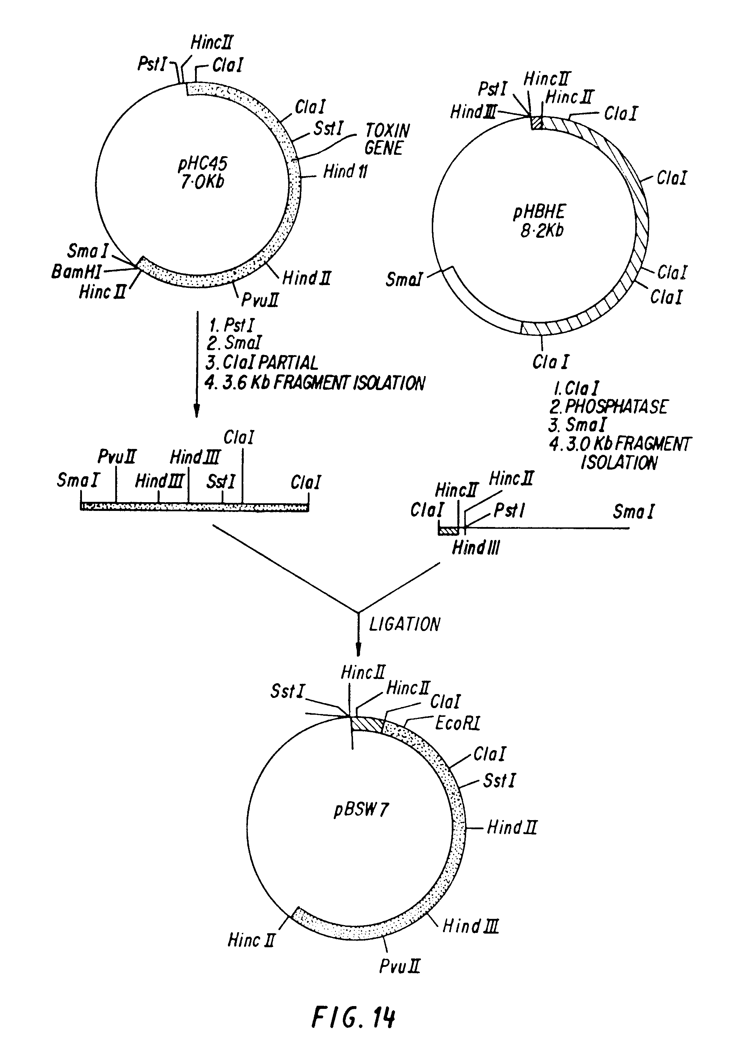

The ligated DNA was introduced into competent cells of E. coli TB-1 strain. After screening by colony hybridization and by studying rapid plasmid preparations as described in earlier Examples, one of the positives was selected, the plasmid isolated from that transformant and mapped for restriction sites. This plasmid is named as pBSW7 and the strain that contains it is named BSW7. The flow chart of constructions is shown in Figure 14.

E IV B. Expression of 130,000 dalton insecticidal protein from BSW7.

E. coli strain BSW7, harboring the recombinant plasmid pBSW7, was grown in LB medium at 30°C for 20 hours. The cells were spun and processed to obtain cell extracts for analyses of proteins as described in earlier examples.

Lanes 6 and 4 in Figure 13 show the proteins from pUC9 and pBSW7 respectively. Figure 13A shows the coomassie blue stained proteins and 138 shows a western blot of a similar gel. The recombinant plasmid pBSW7 expresses a 130,000 dalton protein, similar in size to the protein expressed from pHC45.

E IV C. Insect toxicity assays of extracts of pBSW7.

Cell extracts from BSW7 were prepared as described and toxicity assays were done as described in earlier examples. They were found to be highly toxic to caterpillars.

Example V

Construction of a recombinant plasmid pABS-55 to express B.t. insecticidal protein in B. subtilis.

E V A. The recombinant plasmid pBSW7 described in Example E IV A, was used as the source of the toxin gene. The B. subtilis vector used was pBD64.

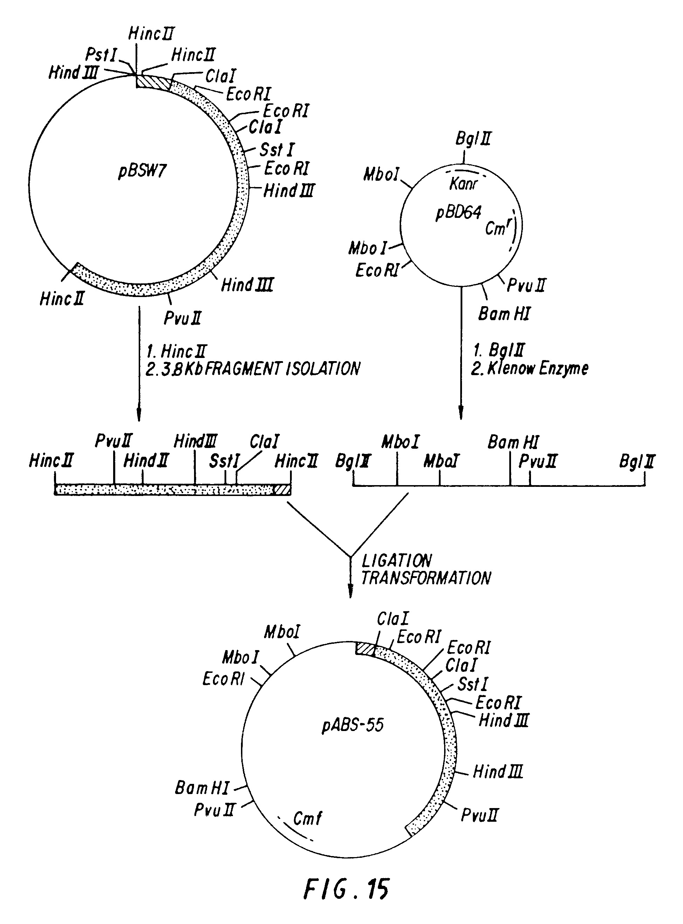

Plasmid pBD64 was digested with the restriction enzyme BglII and the sticky ends generated were filled in using Klenow fragment of DNA polymerase I. pBSW7 was cut with HincII. One part of pBD64 digest was mixed with 4 parts of pBSW7 digest and ligated with T4 DNA ligase at 15°C for 16 hours. The ligated DNA was introduced into the competent cells of B. subtilis strain PSL1 and selected for chloramphenicol resistance. Chloramphenicol resistant colonies were screened for inserts by colony hybridization and for toxin antigen by colony immune blotting. The resultant plasmid is designated as pABS-55 and the strain that harbors this plasmid is named as ABS-55. Sequence of constructions is shown in Figure 15.

E V B. Expression of a 130,000 dalton protein from B. Subtilis strain ABS-55

Cell extracts were made for analysis of proteins from the ABS-55 cells grown in LB medium at 30°C for 20 hours as described in earlier Examples. The proteins were separated on a 8% SDS-polyacrylamide gel. The stained proteins and an immunoblot of the gel are shown in Figures 13A and 13B, respectively. Lanes 2 and 3 show the proteins from pSL1 strain containing pABS-55. These B. subtilis cells make a 130,000 dalton protein that specifically reacts with the antibody for B.t. crystal protein.

E V C. Insect toxicity Assays for cell extracts from ABS-55.

Cell extracts were made from the strain ABS-55 and tested for toxicity as described in earlier Examples. These were found to be highly toxic to insects.

E V D. Formation of a bipyramidal crystal in strain ABS-55.

A culture of ABS-55 was harvested after 22 hours of growth at 30°C and processed for electron micrographic sectioning as described in Methods, supra. Bipyramidal crystals were detected. A picture of an ABS-55 cell containing the crystal is shown in Figure 3D.

It is to be appreciated that various modifications and variations from the specific embodiments of the invention described herein can be made by those of ordinary skill in the art. Such modifications and variations will be apparent from the detailed description hereof and are intended to be fully within the scope of the following claims.

BIBLIOGRAPHY

1. Angus, T.A. (1964) J. Insect pathol., 6, 254,

2. Belyaeva, N.N., and Azizbekyan, R.R. (1968), Virology., 34, 176

3. Burges, H.D., Thomson, E.M. and Latchford, R.A. (1976) J Invertebr. pathol., 27, 87.

4. Cantwell, G.E. and Franklin, B.A. (1966) J. Invertebr. pathol., 30, 131.

5. Dubnau, D. and Davidoff-Abelson, R. (1971) J. Mol. Biol., 56, 209.

6. Gryczan, T., Shivakumar, A.G. and Dubnau, D. (1980) J. Bact., 141, 246.

7. Heimpel, A.M. (1971). In Microbial Control of insects and mites, H.D. Burges and N.W. Hussey (Eds.). Academic press, New York, pp 469-489.

8. Held, G.A., Bulla, L.A., Jr., Farrari, E., Hoch, J., Aronson, A.I. and Minnich, S.A. (1982) Proc. Natn. Acad. Sci. USA, 79, 6065.

9. Horinouchi, S., and Weisblu, B (1982) J. Back., 150, 815

10. Karn, J., Brenner, S., Barnett, L. and Cesareni, G. (1980) Proc. Natn. Acad. Sci. USA., 77, 5172.

11. Klier, A., Fargette, F., Ribier, J. and Rapoport, G. (1982) Embo J., 1, 791.

12. Kronstad, J.W., Schnepf, H.E. and Whiteley, H.R. (1983) J. Bact., 154, 419.

13. Matteuci, M.D. and Caruthers, M.H. (1981) J Am. Chem. Soc., 103, 3185.

14. Messing, J., Crea, R. and Seeburg, P.H. (1981) Nucl. Acid. Res., 9, 309.

15. Morris, O.N. and McErlane, B. (1975) Information Report CC-X-112. Chemical Control Research Institute. Canadian Forestry Service, Ottawa.

16. Ostroff, G.R. and Pene, J.J. (1983) J Bact., 156, 934.

17. Schnepf, H.E. and Whiteley, H.R. (1981) Proc. Natn. Acad. Sci. USA., 78 2893.

18. Sharpe, E.S., Nickerson, K.W., Bulla, L.A., Jr. and Aronson, J.N. (1975) Appl. Microbiol., 30, 1052.

19. Viera, J. and Messing, J. (1982) Gene., 19, 259.

**Claims**

1. A genetically engineered B. subtilis strain which expresses during both the vegetative and the sporulation growth phase a polypeptide of about 135.000 daltons having the immunological properties of a crystal protein of B. thuringiensis, variety Kurstaki HD1 DIPEL, said polypeptide being encoded by a B. thuringiensis DNA fragment which when characterized by digestion with the restriction enzymes ClaI and EcoRI has the following restriction map said DNA fragment being inserted in either orientation in a recombinant plasmid capable of transforming any B. subtilis host species.

2. A recombinant plasmid capable of replication in any B. subtilis host species, said plasmid containing an expressible B. thuringiensis, variety Kurstaki, heterologous DNA fragment which when characterized by digestion with the restriction enzymes ClaI and EcoRI has the follow ing restriction map said DNA fragment being inserted in either orientation and coding for a polypeptide of about 135.000 daltons which has the immunological properties of a crystal protein of B. thuringiensis variety Kurstaki, said plasmid further including an expression mechanism for said heterologous DNA which is recognized by the host species.

3. The genetically engineered B. subtilis strain ABS-55 (on deposit at American Type Culture Collection under accession number 53122) which expresses during both the vegetative and the sporulation growth phase a polypeptide of about 130.000 daltons having the immunological properties of a crystal protein of B. thuringiensis, variety Kurstaki, said polypeptide being encoded by a B. thuringiensis DNA fragment which when characterized by digestion with the restriction enzymes ClaI and EcoRI has the following restriction map said DNA fragment being inserted in a recombinant plasmid capable of transforming any B. subtilis host species.

4. A recombinant plasmid capable of replication in any B. subtilis host species, said plasmid containing an

16

expressible B. thuringiensis, variety Kurstaki, heterologous DNA fragment which when characterized by digestion with the restriction enzymes ClaI and EcoRI has the following restriction map said DNA fragment coding for a polypeptide of about 130.000 daltons which has the immunological properties of a crystal protein of B. thuringiensis, variety Kurstaki, said plasmid further including an expression mechanism for said heterologous DNA which is recognized by the host species.

5. A process for producing B. thuringiensis, variety Kurstaki, delta-endotoxin proteins comprising expressing the B. subtilis strain, according to Claim 1 or 3 during its vegetative phase.

**Revendications**

1. Une souche de B.subtilis manipulée génétiquement qui exprime durant l'une et l'autre phases de croissance, végétative et sporulation, un polypeptide d'environ 135.000 daltons ayant les propriétés immunologiques d'une protéine cristal de B.thuringiensis, variété Kurstaki HD1 DIPEL, ledit polypeptide étant encodé par un fragment d'ADN de B.thuringiensis qui, lorsqu'il est caractérisé par digestion avec les enzymes de restriction ClaI et EcoRI, a la carte de restriction suivante ledit fragment d'ADN étant inséré dans l'une ou l'autre orientation dans un plasmide recombinant capable de transformer une espèce de B.subtilis hôte quelconque.

2. Un plasmide recombinant capable de réplication dans une espèce de B.subtilis hôte quelconque, ledit plasmide contenant un fragment d'ADN hétérologue de B.thuringiensis, variété Kurstaki, expressible qui lorsqu'il est caractérisé par digestion avec les enzymes de restriction ClaI et EcoRI a la carte de restriction suivante ledit fragment d'ADN étant inséré dans l'une ou l'autre orientation et codant pour un polypeptide d'environ 135.000 daltons qui a les propriétés immunologiques d'une protéine cristal de B.thuringiensis variété Kurstaki, ledit plasmide comprenant de plus un mécanisme d'expression pour ledit ADN hétérologue qui est reconnu par l'espèce hôte.

3. La souche de B.subtilis manipulée génétiquement ABS-55 (déposée à l'American Type Culture Collection et enregistrée sous le numéro 53122) qui exprime durant l'une et l'autre phase de croissance, végétative et sporulation, un polypeptide d'environ 130.000 daltons ayant les propriétés immunologiques d'une protéine cristal de B.thuringiensis, variété Kurstaki, ledit polypeptide étant encodé par un fragment d'ADN de B.thuringiensis qui lorsqu'il est caractérisé par digestion avec les enzymes de restriction ClaI et EcoRI a la carte de restriction suivante

```
        EcoRI        ClaI
  ClaI  |      EcoRI |   EcoRI
    |   |        |   |     |
────┴───┴────────┴───┴─────┴──────────
```

ledit fragment d'ADN étant inséré dans un plasmide recombinant capable de transformer une espèce de B.subtilis hôte quelconque.

4. Un plasmide recombinant capable de réplication dans une espèce de B.subtilis hôte quelconque, ledit plasmide contenant un fragment d'ADN hétérologue de B.thuringiensis, variété Kurstaki, expressible qui lorsqu'il est caractérisé par digestion avec les enzymes de restriction ClaI et EcoRI a la carte de restriction suivante

```
        EcoRI        ClaI
  ClaI  |      EcoRI |   EcoRI
    |   |        |   |     |
────┴───┴────────┴───┴─────┴──────────
```

ledit fragment d'ADN codant pour un polypeptide d'environ 130.000 daltons qui a les propriétés immunologiques d'une protéine cristal de B.thuringiensis, variété Kurstaki, ledit plasmide comprenant en plus un mécanisme d'expression pour ledit ADN hétérologue qui est reconnu par l'espèce hôte.

5. Un procédé pour la production de protéines delta-endotoxine de B.thuringiensis, variété Kurstaki, consistant à faire exprimer la souche de B.subtilis selon la revendication 1 ou 3 pendant sa phase végétative.

## Patentansprüche

1. Gentechnisch veränderter Stamm von B. subtilis, der sowohl in der vegetativen als auch in der Sporulations-Wachstumsphase ein Polypeptid von etwa 135.000 Dalton exprimiert und die immunologischen Eigenschaften eines Kristallproteins von B. thuringiensis, Varietät Kurstaki HD1 DIPEL, aufweist, wobei für das Polypeptid ein B. thuringiensis-DNS-Fragment kodiert, das, wenn durch Verdauung mit den Restriktionsenzymen ClaI und EcoRI charakterisiert, die folgende Restriktionskarte zeigt

```
          EcoRI           ClaI            ClaI
  ClaI    |       EcoRI   |        ClaI   |              ClaI
    |     |         |     |         |     |               |
────┴─────┴─────────┴─────┴─────────┴─────┴───────────────┴──
```

wobei das DNS-Fragment in beliebiger Orientierung in ein rekombinantes Plasmid insertiert ist, das jede B. subitilis-Wirtsspezies transformieren kann.

2. Rekombinantes Plasmid, das zur Replikation in jeder B. subtilis-Wirtsspezies fähig ist, welches Plasmid ein exprimierbares heterologes DNS-Fragment von B. thuringiensis, Varietät Kurstaki, enthält, welches, wenn durch Verdauung mit den Restriktionsenzymen ClaI und EcoRI charakterisiert, die folgende Restriktionskarte zeigt

```
          EcoRI           ClaI            ClaI
  ClaI    |       EcoRI   |        ClaI   |              ClaI
    |     |         |     |         |     |               |
────┴─────┴─────────┴─────┴─────────┴─────┴───────────────┴──
```

wobei das DNS-Fragment in beliebiger Orientierung insertiert ist und für ein Polypeptid von etwa 135.000 Dalton kodiert, das die immunologischen Eigenschaften eines Kristallproteins von B. thuringiensis, Varietät Kurstaki, aufweist, und welches Plasmid weiterhin einen Expressionsmechanismus für die heterologe DNS einschließt, der von der Wirtsspezies erkannt wird.

3. Gentechnisch veränderter B. subtilis-Stamm ABS-55 (deponiert bei der American Type Culture Collection unter der Zugangsnummer 53122), der sowohl während der vegetativen als auch während der Sporulations-Wachstumsphase ein Polypeptid von etwa 130.000 Dalton mit den immunologischen Eigenschaften eines Kristallproteins von B. thuringiensis, Varietät Kurstaki, exprimiert, welches Polypeptid von einem B. thuriengiensis-DNS-Fragment kodiert wird, das, wenn durch Verdauung mit den Restriktionsenzymen ClaI und EcoRI charakterisiert, die folgende Restriktionskarte aufweist wobei das DNS-Fragment in ein rekombinantes Plasmid insertiert ist, das jede B. subtilis-Wirtsspezies transformieren kann.

4. Rekombinantes Plasmid, das zur Replikation in jeder B. subtilis-Wirtsspezies fähig ist, welches Plasmid ein exprimierbares heterologes DNS-Fragment von B. thuringiensis, Varietät Kurstaki, enthält, das, wenn durch Verdauung mit den Restriktionsenzymen ClaI und EcoRI charakterisiert, die folgende Restriktionskarte aufweist wobei das DNS-Fragment für ein Polypeptid von etwa 130.000 Dalton kodiert, das die immunologischen Eigenschaften eines Kristallproteins von B. thuringiensis, Varietät Kurstaki, aufweist, welches Plasmid weiterhin einen Expressionsmechanismus für die heterologe DNS einschließt, der von der Wirtsspezies erkannt wird.

5. Verfahren zur Erzeugung von Delta-Endotoxin-Proteinen von B. thuringiensis, Varietät Kurstaki, durch Exprimieren des B. subtilisstamms nach Anspruch 1 oder 3 während seiner vegetativen Phase.

FIG. 1

FIG.2A

FIG.2B

FIG.3A  1.0 μm
FIG.3B
FIG.3C
FIG.3D
FIG.3E
FIG.3F

FIG.4

FIG.5A

FIG.5B

FIG. 6

FIG.7B

FIG.7A

FIG. 8

FIG.9A

FIG.9B

FIG.10A

FIG.10B

1.0 μm

FIG.10C

FIG.10D

FIG.10E

EP 0 202 470 B1

FIG.IIB

FIG.IIA

FIG.IIC

1.0 μm

FIG. 12

EP 0 202 470 B1

FIG.13A

FIG.13B

FIG. 14

FIG. 15